# EUROPÄISCHE PATENTSCHRIFT

(11) **EP 0 469 377 B1**
(45) Veröffentlichungstag und Bekanntmachung des Hinweises auf die Patenterteilung: **24.04.1996**
(21) Anmeldenummer: 91111830.5
(22) Anmeldetag: 16.07.1991
(51) Int. Cl.: G01N 33/543, G01N 21/55

(54) **Analysesystem und Verfahren zur Bestimmung eines Analyten in einer fluiden Probe**
Analysis system and method for the determination of an analyte in a liquid sample
Dispositif et procédé pour la détermination d'une analyte dans un échantillon liquide

(30) Priorität: 02.08.1990 DE 4024476
(43) Veröffentlichungstag der Anmeldung: 05.02.1992
(73) Patentinhaber: BOEHRINGER MANNHEIM GMBH, D-68298 Mannheim (DE)
(72) Erfinder: Knoll, Wolfgang, W-6500 Mainz 1 (DE); Schmitt, Franz-Josef, W-5501 Leiwen (DE)
(74) Vertreter: Pfeifer, Hans-Peter, Dr. Patentanwalt

(56) Entgegenhaltungen:
- EP-A- 0 353 937
- WO-A-90/05305
- US-A- 4 889 427
- US-A- 4 992 385
- SPIE, Band 954, 1988, Redondo Beach, CA (US); SADOWSKI, Seiten 413-419/
- JOURNAL OF APPLIED PHYSIOLOGY, Band 67, Nr. 8, 15. April 1990; HICKEL et al., Seiten 3572-3575/
- NATURE, Band 332, 14. April 1988; ROTHENHÄUSLER et al., Seiten 615-617/
- MRS BULLETIN, Band 16, Nr. 7, 1991; Seiten 29-39/

## Beschreibung

Die Erfindung betrifft ein Analysesystem und ein Verfahren zur Bestimmung eines Analyten in einer fluiden Probe, insbesondere einer Körperflüssigkeit mittels einer spezifischen Bindungsreaktion zweier bioaffiner Bindungspartner.

Bei der analytischen Untersuchung von Proben, insbesondere im medizinischen Bereich, haben Analyseverfahren, die auf der spezifischen Bindungsreaktion zweier bioaffiner Bindungspartner beruhen, zunehmend an Bedeutung gewonnen. Spezifische Bindungsreaktionen in diesem Sinne sind insbesondere immunologische Interaktionen, also Wechselwirkungen zwischen Antigenen bzw. Haptenen einerseits und Antikörpern andererseits. Es können jedoch auch andere spezifische bioaffine Wechselwirkungen verwendet werden, wie Lektin-Zucker, eine Wirkstoff-Rezeptor-Wechselwirkung oder die spezifische Bindung zwischen Biotin und Streptavidin. Im folgenden wird einfachheitshalber, jedoch ohne Beschränkung der Allgemeinheit, auf immunologische Bindungsreaktionen Bezug genommen.

Es sind eine Vielzahl von verschiedenen immunologischen Analyseverfahren bekannt. Vielfach wird dabei einer der Bindungspartner an einem Träger fixiert und im Verlauf der Analyse mit dem anderen Bindungspartner in Kontakt gebracht, wobei der Grad der Bindung des freien Bindungspartners an den trägerfixierten Bindungspartner ein Maß für die Analyse ist. Dabei kann der zu bestimmende Analyt selbst der freie Bindungspartner sein. Der freie Bindungspartner kann aber auch ein Bestandteil des Reagenzsystems sein, welcher direkt oder indirekt mit dem Analyten wechselwirkt, so daß seine Bindung an den festen Bindungspartner für die Konzentration des Analyten charakteristisch ist. Die Erfindung bezieht sich unabhängig vom spezifischen Verfahrensablauf allgemein auf Analyseverfahren, bei denen die Bindungsreaktion zwischen einem trägerfixierten Bindungspartner und einem freien Bindungspartner ein Maß für die Bindeaktivität des Analyten in der Probe ist.

Bei derartigen Verfahren wird der Nachweis der Bindung des freien Bindungspartners an den festphasenfixierten Bindungspartner üblicherweise dadurch ermöglicht, daß der freie Bindungspartner mit einem Markierungsbestandteil ("Label") markiert ist. Gebräuchlich ist insbesondere die Markierung mit einem Enzym oder mit einem fluoreszierenden Bestandteil. Die dadurch ermöglichte indirekte Beobachtung der Bindung erlaubt Analysen mit hoher Spezifität und Empfindlichkeit, hat jedoch auch erhebliche Nachteile. Insbesondere erfordert der Nachweis der Fluoreszenzmarkierung einen erheblichen apparativen Aufwand. Der Nachweis eines Enzyms erfordert einen speziellen Reaktionsschritt, durch den die Analysedauer verlängert und der Reaktionsablauf komplizierter wird.

Insoweit sind Verfahren, die eine unmittelbare Beobachtung der Bindung eines freien Bindungspartners an einen trägerfixierten Bindungspartner erlauben, überlegen. Insbesondere ist vorgeschlagen worden, durch reflexionsoptische Techniken die Zunahme der Schichtdicke einer extrem dünnen Schicht mit dem trägerfixierten Bindungspartner durch die Bindung des freien Bindungspartners zu beobachten. Ein Überblick über diese Techniken wird gegeben in J. W. Sadowski: "Review of optical methods in immunosensing", SPIE, Vol. 954 Optical testing and Metrology II(1988),413-419.

Ein Analysesystem auf dieser Basis besteht allgemein aus Analyseelementen und einem hierauf spezifisch abgestimmten Auswertegerät. Die Analyseelemente haben einen Testbereich, der mit der Probe kontaktiert wird und in dem der erste Bindungspartner in extrem dünner Schichtstärke trägerfixiert ist. Das Auswertegerät hat eine reflexionsoptische Meßeinrichtung zur Erfassung einer aus der Bindung des zweiten Bindungspartners an den ersten Bindungspartner resultierenden Schichtdickenänderung und eine üblicherweise mikroprozessorgesteuerte Auswerteeinheit zur Ermittlung des Analysewertes aus der Schichtdickenänderung. Wegen weiterer Einzelheiten wird auf den zitierten Artikel und die darin referierten Originalarbeiten Bezug genommen.

Eines der reflexionsoptischen Verfahren, die für immunologische Analysen vorgeschlagen wurden, ist die Oberflächenplasmonenresonanz, im folgenden kurz als SPR (surface plasmon resonance) bezeichnet. Dabei besteht das Analyseelement, welches vielfach auch als "optischer Immunsensor" bezeichnet wird, aus einem durchsichtigen dielektrischen Material, auf dem in sehr geringer Schichtstärke (typischerweise 50 nm) eine metallisch leitende Schicht aufgebracht ist, welche ihrerseits direkt oder indirekt den trägerfixierten Bindungspartner trägt. Um die Anregung von Oberflächenplasmonen zu ermöglichen, ist die metallisierte Oberfläche üblicherweise entweder Bestandteil eines Prismas oder die metallisierte Oberfläche ist als optisches Gitter strukturiert. Eine solche zur Erzeugung von Oberflächenplasmonen geeignete Anordnung wird im folgenden zusammenfassend als "Plasmonenkoppleranordnung" bezeichnet.

Das Analyseelement kann mit dem Auswertegerät fest verbunden sein. Bevorzugt werden jedoch auswechselbare einmalverwendbare (disposible) Analyseelemente eingesetzt. Im Falle der SPR läßt sich ein für eine Plasmonenkoppleranordnung erforderliches optisches Gitter kostengünstig und präzise mit Hilfe des "injection-molding"-Verfahrens herstellen.

Es sind bereits eine Reihe optischer Immunsensoren vorgeschlagen worden. Verwiesen sei insbesondere auf die EP-A-0 112 721, EP-A-0 276 142, US-A-4 889 427, in denen eine Vielzahl von geeigneten Analyseverfahren beschrieben werden, sowie auf die EP-A-0 254 575, die auf Einzelheiten der Erzeugung geeigneter Gitterstrukturen eingeht. Mit apparativen Fragen der reflexionsoptischen Meßauswertung befaßt sich beispielsweise die EP-A-0 341 927.

Ein besonderes Problem der bekannten optischen Immunsensoren besteht darin, daß die Meßgenauigkeit durch unspezifische Bindungen wesentlich beeinträchtigt wird. Da die genannten reflexionsoptischen Messungen lediglich die Schichtstärke im Meßbereich undifferenziert erfassen, führt jede unspezifische (für die Analyse nicht charakteristische) Bindung irgendwelcher Bestandteile der Probenflüssigkeit in dem Testbereich zu einer Verfälschung des Meßergebnisses.

Der Erfindung liegt die Aufgabe zugrunde, die Genauigkeit optischer Immunsensoren, insbesondere hinsichtlich der Meßfehler durch unspezifische Bindungen, zu verbessern.

Die Aufgabe wird durch ein Analysesystem gemaß Anspruch 1 und durch ein Verfahren gemaß Anspruch 10 gelöst.

Der Testbereich ist so ausgebildet, daß eine darauf aufgegebene Probe die verschiedenen Teilbereiche kontaktiert, so daß eine Vergleichsmessung möglich ist. Weiter ist wesentlich, daß die Teilbereiche bezüglich unspezifischer Bindungen des zweiten Bindungspartners ähnliche Eigenschaften haben. Dadurch, daß die Dickenänderung in den verschiedenen Teilbereichen mit Hilfe der reflexionsphotometrischen Meßeinrichtung getrennt erfaßt und miteinander verglichen wird, können die Einflüsse unspezifischer Bindungen eliminiert werden. Zugleich gibt die räumlich differenzierte reflexionsphotometrische Messung vielfach die Möglichkeit, Oberflächenfehler, die die Auswertung stören könnten, zu eliminieren.

Der Begriff "Bindeaktivität" bezieht sich allgemein auf die Fähigkeit des in den Teilbereichen fixierten Bindungspartners, den freien Bindungspartner zu binden. Sie laßt sich auf verschiedene Weise beeinflussen. Die Konzentration des trägerfixierten Bindungspartners kann unterschiedlich sein. Die Bindeaktivität kann aber auch von anderen Einflußgrößen, insbesondere der aus sterischen Gründen unterschiedlichen Erreichbarkeit der Bindungsstellen abhängen. Die Bindeaktivität in den Teilbereichen kann sich sowohl bezüglich der maximalen Bindekapazität, als auch kinetisch, also bezüglich der Bindegeschwindigkeit, unterscheiden.

Die Teilbereiche können grundsätzlich mit Abstand voneinander in dem Testbereich des Analyseelements angeordnet sein. Aus meßtechnischen und herstellungstechnischen Gründen ist es jedoch bevorzugt, wenn sie unmittelbar aneinandergrenzen.

Im allgemeinsten Fall kann der Testbereich zwei oder einige wenige Teilbereiche aufweisen, die sich jeweils voneinander bezüglich der Bindeaktivität des trägerfixierten Bindungspartners unterscheiden, jedoch ähnliche Eigenschaften bezüglich unspezifischer Bindungen haben. Bevorzugt ist eine Ausführungsform, bei der der Testbereich eine Vielzahl von Teilbereichen unterschiedlicher Bindeaktivität aufweist, die sich zwei oder mehreren Sätzen von Teilbereichen zuordnen lassen, wobei im Regelfall jeder Satz mehrere Teilbereiche umfaßt. Dabei haben die Teilbereiche des gleichen Satzes jeweils die gleiche Bindeaktivität des ersten Bindungspartners und die Teilbereiche verschiedener Sätze unterschiedliche Bindeaktivitäten des ersten Bindungspartners. Vorzugsweise sind die Teilbereiche verschiedener Sätze in dem Testbereich alternierend angeordnet, wobei eine regelmäßig alternierende Anordnung (A,B,C, A,B,C, A,B,...) bevorzugt, jedoch nicht notwendig ist.

Die Erfindung wird im folgenden anhand eines in den Figuren schematisch dargestellten Ausführungsbeispiels näher erläutert. Es zeigen:
- Fig. 1: ein erfindungsgemäßes Analysesystem in perspektivischer Ansicht,
- Fig. 2: eine Prinzipdarstellung zur Erläuterung des bei der Erfindung angewandten Meßverfahrens,
- Fig. 3: einen vergrößerten Ausschnitt aus Fig. 2,
- Fig. 4: eine Aufsicht auf einen Testbereich,
- Fig. 5: eine graphische Darstellung der Winkelabhängigkeit des gemessenen Intensitätssignals für zwei verschiedene Teilbereiche, jeweils vor und nach der spezifischen Bindungsreaktion,
- Fig. 6: den zeitlichen Verlauf des Signalminimums aus Fig. 5 für zwei verschiedene Teilbereiche.
- Fig. 7: eine Aufsicht auf eine weitere Ausführungsform eines Analyseelementes.

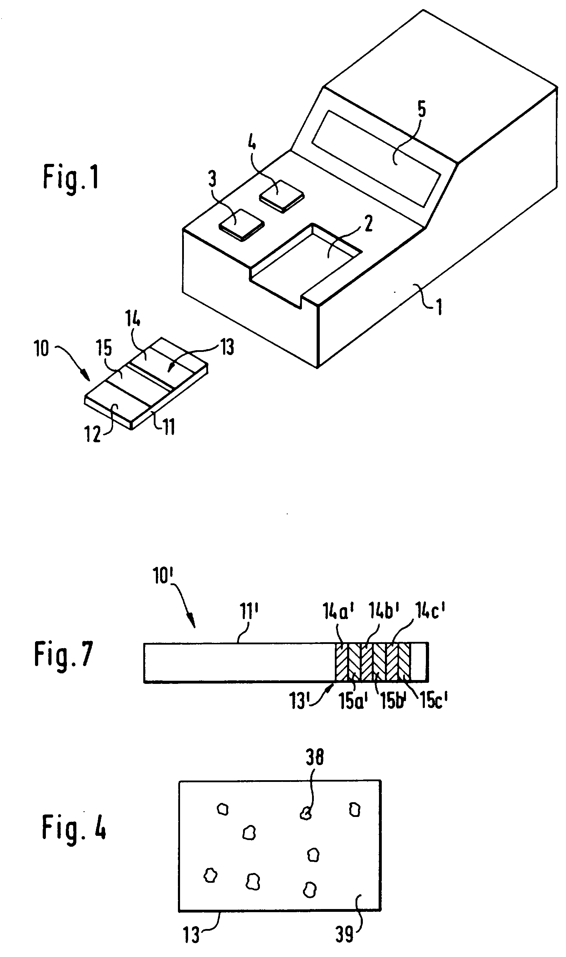

Das in Fig. 1 dargestellte Analysesystem besteht aus einem Auswertegerät 1 und Testträgern 10, von denen in der Figur nur einer dargestellt ist.

Das Auswertegerät 1 hat einen Adapter 2 zur Aufnahme eines Testträgers 10, Eingabeelemente, beispielsweise in Form von Tasten 3,4 und eine Einrichtung zur Ausgabe von Analyseergebnissen, beispielsweise ein Display 5.

Das Analyseelement 10 hat eine Tragschicht 11, auf deren im Bild oberen Oberfläche 12 ein Testbereich 13 vorgesehen ist, der im vorliegenden Falle aus zwei Teilbereichen 14 und 15 besteht, welche eng benachbart sind.

Zur Benutzung wird das Analyseelement 10 in den Adapter 2 eingelegt und die Probe wird derartig auf den Testbereich 13 aufgebracht, daß sie mit beiden Teilbereichen 14 und 15 in Kontakt steht. Um dies zu erleichtern, hat der Testbereich 13 vorzugsweise eine geringe Flächenausdehnung von typischerweise weniger als 6x6 mm.

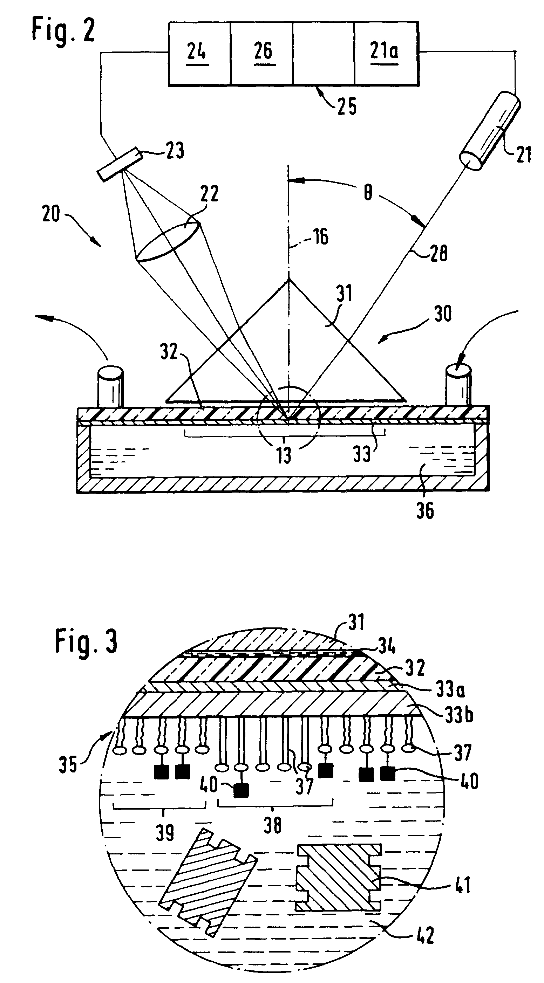

Fig. 2 zeigt in einer stark abstrahierten Darstellung Einzelheiten einer geeigneten Meßanordnung anhand eines Labormuster.

Die dargestellte reflexionsoptische Meßeinrichtung 20 ist zur Oberflächenplasmonenmikroskopie ausgebildet. Sie besteht im wesentlichen aus einem Laser 21 einschließlich elektronischer Ansteuerungseinheit 21a, einer Sammellinse 22, einem CCD-Array 23 und einer Bildanalyseeinrichtung 24. Die Ansteuereinheit 21a und die Bildanalyseeinrichtung 24 sind Bestandteile der insgesamt mit 25 bezeichneten zweckmaßigerweise mikroprozessorgesteuerten elektronischen Schaltung des Gerätes, die außerdem eine Auswerteeinheit 26 zur Auswertung der Meßsignale enthält. Die Auswerteeinheit 26 enthält u.a. eine Vergleichseinheit zum Vergleich der an den Teilbereichen 14,15 des Testbereichs 13 gewonnenen Meßsignale.

Der von dem Laser 21 ausgehende Primärstrahl 28 fällt unter einem Winkel θ zur Flächennormalen 16 des Testbereiches 10 ein. Das reflektierte Licht wird von der Linse 22 auf das in der Bildebene angeordnete CCD-Array abgebildet.

Um in dem Testbereich 13 Plasmonen anzuregen, ist eine insgesamt mit 30 bezeichnete Plasmonenkoppleranordnung vorgesehen, welche im dargestellten Fall aus einem Prisma 31 in Kretschmann-Anordnung, einer dielektrischen optisch transparenten Tragschicht 32 und einer dünnen Metallschicht 33 besteht, die auf der von dem Prisma 31 abgewandten Seite der Tragschicht 32 aufgedampft ist.

Der Schichtaufbau ist in Fig. 3 deutlicher zu erkennen, welche eine vergrößerte Ausschnittsdarstellung von Fig. 2 zeigt. Dabei ist auch eine dünne Schicht einer sogenannten Indexflüssigkeit 34 zu erkennen, die den gleichen Brechungsindex wie das Prisma 31 und die Tragschicht 32 hat und diese beiden Teile ohne optische Brechung verbindet. Außerdem erkennt man in Fig. 3, daß die Metallschicht 33 bei der dargestellten bevorzugten Ausführungsform aus einer dünnen Unterschicht 33a aus Chrom und einer etwas dickeren oberen Schicht 33b aus Gold besteht.

Bei dem in Fig. 2 dargestellten Labormuster ist die Tragschicht 32 Teil einer Küvette 36, durch die eine Probenflüssigkeit in der in Fig. 2 durch Pfeile angedeuteten Weise geleitet werden kann. Dies ist für grundlegende Untersuchungen der der Erfindung zugrundeliegenden Verfahrensweise zweckmäßig. Bei einer kommerziellen Ausführungsform wird das Analyseelement 10 mit seiner Tragschicht 11 auf das Prisma 31 derartig aufgelegt, daß sein Meßbereich 13 von dem Primärstrahl 28 des Lasers 21 beleuchtet wird. Vorzugsweise wird für die Plasmonenkoppleranordnung statt des Prismas 31 eine optische Gitterstruktur verwendet, welche durch "injection molding" in die Tragschicht 11 des Analyseelementes 10 eingeprägt ist.

Einzelheiten der Oberflächenplasmonenresonanz-Technik sind, wie einleitend dargelegt, bekannt. Es genügen deshalb einige grundlegende Erläuterungen zur Funktionsweise der dargestellten Meßvorrichtung.

Oberflächenplasmonen sind kollektive Anregungszustände der Elektronen in einem Metall, hier der Metallschicht 33. Sie können mittels einer Plasmonenkoppleranordnung resonant durch Laserlicht angeregt werden. Resonanz zwischen dem eingestrahlten Licht und den Plasmonen tritt ein, wenn die diesbezügliche Resonanzbedingung erfüllt ist, wonach die Energie und der Impuls der P-polarisierten, flächenparallelen Komponente des eingestrahlten Lichts gleich der Energie und dem Impuls der Oberflächenplasmonen ist.

Die SPR stellt ein empfindliches Instrument zum Studium extrem dünner Schichtstärken dar, weil der Plasmonenimpuls (und damit bei gegebenem Primärlichtstrahl der Resonanzwinkel ϑ) in kritischer Weise von den Eigenschaften der Metalloberfläche, insbesondere dem Brechungsindex und der Dicke einer auf der Metallschicht vorhandenen Beschichtung abhängt. Deswegen ermöglicht die SPR eine hochempfindliche Schichtdickenanalyse.

Während die Oberflächenplasmonenresonanz (SPR) lediglich eine integrierte Beobachtung des von dem Laserstrahl beleuchteten Meßbereichs ermöglicht, erlaubt die Oberflächenplasmonenmikroskopie (SPM) eine räumlich differenzierte Charakterisierung der Schichtdickenverteilung. Einzelheiten dieser Technik sind den folgenden Publikationen zu entnehmen:
- DE-A-37 20 387
- B. Rothenhäusler, W. Knoll
   "Surface-plasmon microscopy" Nature, Vol. 332, No. 6165, pp.615-617 (1988)
- W. Hickel, B. Rothenhäusler, W. Knoll:
   "Surface plasmon microscopic characterization of external surfaces", J.Appl.Phys. 66 (1989) pp.4832-4836
- W. Hickel, W. Knoll
   "Surface plasmon optical characterization of lipid monolayers at 5µm lateral resolution", J.Appl.Phys. 67 (1990) 3572ff.

Charakteristisch für die SPM ist, daß das reflektierte Licht mit Hilfe einer Linse auf eine Bildebene abgebildet wird. Mit anderen Worten wird das reflektierte Licht Fourier-rückkonvertiert in den Ortsraum. Die dadurch erzeugte Abbildung der Probenfläche kann auf verschiedene Weise beobachtet werden. Beispielsweise kann ein lichtempfindliches Element in der Bildebene bewegt werden, um an verschiedenen Positionen die reflektierte Intensität abzutasten. Vorzugsweise wird jedoch eine zweidimensional ortauflösende lichtempfindliche Anordnung, beispielsweise ein CCD-Array in der Bildebene positioniert und die erhaltenden Signale werden mit üblichen bildanalytischen Verfahren ausgewertet.

Weitere Einzelheiten sind in den genannten Arbeiten beschrieben, in denen die SPM zur Charakterisierung verschiedener Schichtstrukturen eingesetzt wird. Die Verwendung für chemisch-analytische Zwecke wird darin nicht angesprochen.

Der Testbereich mit den Teilbereichen unterschiedlicher Bindeaktivität kann insbesondere mit Hilfe einer Langmuir-Blodgett-Technik (LB-Technik) präpariert werden. Diese Technik ist bekannt (z.B. H. Kuhn, D. Möbius, H. Bücher in: "Physical Methods of Chemistry" A. Weissberger und B.W. Rossiter, Edit., Wiley-Interscience, New York (1972) Vol.1) und wurde auch in den zitierten Arbeiten eingesetzt, um Lipid-Monoschichten zu erzeugen. Dabei wird die natürliche Tendenz amphiphiler, d.h. oberflächenaktiver, organischer Moleküle, sich an einer Wasser-Luft-Grenzfläche selbst zu organisieren, ausgenutzt. Eine Lösung einer solchen Substanz wird auf einer reinen Wasseroberfläche ausgebreitet ("gespreitet"), wobei der polare Teil des Moleküls, die sogenannte hydrophile Kopfgruppe, zum Wasser hin gerichtet ist, während der unpolare Teil (typischerweise eine hydrophobe Kohlenwasserstoffkette) zur Luft hin orientiert ist. Die bei dem Spreiten auf der Wasseroberfläche spontan gebildete monomolekulare Schicht läßt sich mittels einer beweglichen Barriere komprimieren.

Mit einem solchen Verfahren läßt sich, wie im Rahmen der vorliegenden Erfindung festgestellt wurde, ein Testbereich erzeugen, der aus einer Monoschicht von Molekülen besteht, an denen ein bioaffiner Bindungspartner fixiert ist. Dabei bildet die Monoschicht Teilbereiche mit höherer und niedrigerer Bindeaktivität des Bindungspartners im Sinne der vorliegenden Erfindung. Im einzelnen wird dazu folgendermaßen vorgegangen.

Auf einer reinen Wasseroberfläche wird eine Lipid-Verbindung, beispielsweise Dimyristoylphosphatidyläthanolamin (DMPE) gespreitet, wobei der Lösung ein verhältnismäßig geringer Teil (typischerweise etwa 5%) des Lipids beigemischt ist, an das der jeweilige Bindungspartner (beispielsweise durch kovalente Bindung über eine Spacer-Gruppe an die Kopfgruppe des Lipid-Moleküls) gebunden ist. Wenn der Bindungspartner beispielsweise Biotin ist, wird ein entsprechend biotinyliertes Lipid (im konkreten Fall biotinyliertes DMPE) eingesetzt.

Bei der Kompression einer solchen Monoschicht auf der Wasseroberfläche entstehen zwei (zweidimensionale) Phasen, nämlich kristalline Bereiche in einer fluid-amorphen Matrix. Anhand von Druck-Flächendiagrammen läßt sich ein breiter Koexistenzbereich dieser beiden Phasen feststellen. Die beiden Phasen weisen gegenüber dem Bindungspartner Streptavidin eine unterschiedliche Bindeaktivität auf.

Der auf der Wasseroberfläche gebildete LB-Monofilm kann durch einfaches Ein- und Austauchen auf eine feste Tragschicht übertragen werden. Durch Vielfach-Tauchen lassen sich auch Multischichten aufbauen. Im Rahmen der vorliegenden Erfindung ist jedoch eine sehr geringe Schichtstärke, vorzugsweise eine Monoschicht, vorteilhaft.

Nach dem Austauchen bilden die hydrophoben Kettenenden an Luft einen stabilen Abschluß des LB-Films. Die Stabilität läßt sich dadurch verbessern, daß polymerisierbare Lipide verwendet werden. Damit läßt sich sowohl eine Quervernetzung innerhalb der Lipidschicht, als auch eine kovalente Verknüpfung der polaren Kopfgruppen zum Substrat erreichen.

Bei der Analyse wässriger Proben ist es erforderlich, den LB-Film nach der Übertragung auf die Tragschicht ständig in Kontakt mit einer wässrigen Phase zu halten. Es besteht jedoch die Möglichkeit, sie durch Herstellung kovalenter Verbindungen (z.B. durch nachträgliches Polymerisieren) zu stabilisieren.

Die Struktur eines solchen Testbereichs ist in Fig. 3 symbolisch dargestellt. Die Lipidmoleküle 37 bilden eine Lipid-Monoschicht 35. Sie sind in den kristallinen Teilbereichen 38 streng geordnet (in der Figur wird die Ordnung durch gerade Linien an den Moleküldarstellungen symbolisiert), während sie in den fluid-amorphen Teilbereichen weniger geordnet sind (in der Figur durch gewellte Linien dargestellt). Die kristallinen Teilbereiche 38 weisen eine geringere Bindeaktivität gegenüber Streptavidin als die fluid-amorphen Teilbereiche 39 auf.

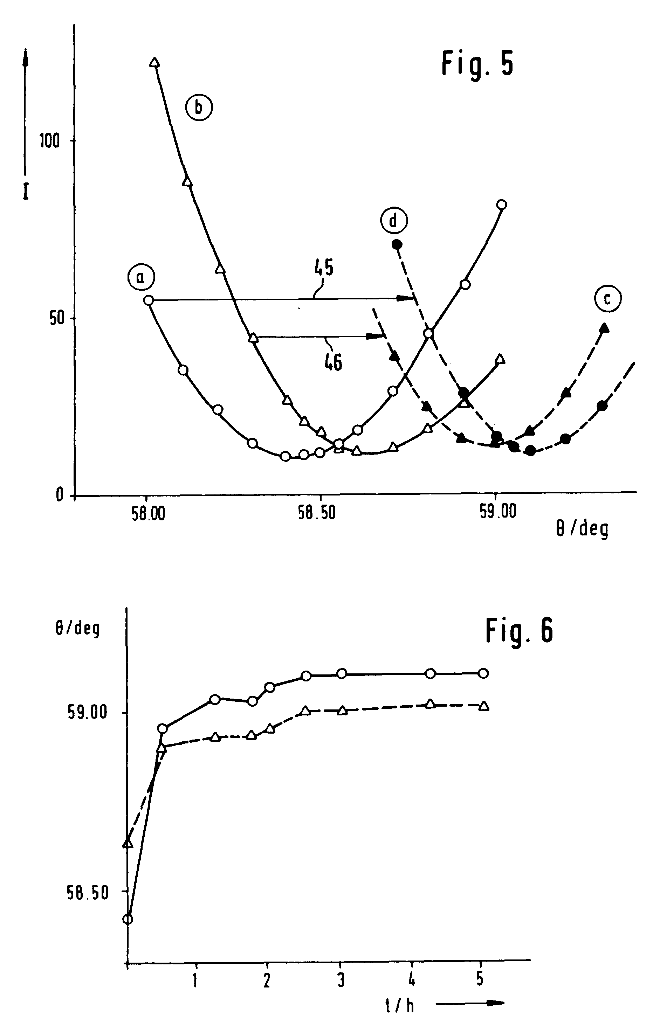

Die Anordnung der Teilbereiche 38,39 in dem Testbereich 13 ist in Fig. 4 in einer nicht maßstäblichen Prinzipdarstellung gezeigt. Die kristallinen Teilbereiche 38 sind inselartig in der fluid-amorphen-Matrix verteilt. In diesem Fall hat der Testbereich 13 also eine Vielzahl von Teilbereichen 38 mit geringerer Bindeaktivität des trägerfixierten Bindungspartners und einen zusammenhängenden Teilbereich 39 mit einer hohen Bindeaktivität des trägerfixierten Bindungspartners.

Infolge dieser unterschiedlichen Bindeaktivität des Biotins (fixierter Bindungspartner) in den Teilbereichen 38 und 39 wird das hiermit spezifisch bindungsfähige Streptavidin 41 (freier Bindungspartner), welches in einer Probenflüssigkeit 42 enthalten ist, in dem Teilbereich 39 in stärkerem Maße spezifisch gebunden als in den kristallinen Teilbereichen 38. Im Gegensatz dazu ist die unspezifische Bindung an der als Substrat für die Fixierung des ersten Bindungspartners dienenden Lipid-Monoschicht 35 überall praktisch gleich ist.

Hierdurch läßt sich die spezifische Bindungsreaktion exakt und unter weitgehender Elimination von durch unspezifische Bindungen verursachten Meßfehlern auswerten. Dabei kann wie folgt vorgegangen werden.

Vor Beginn und während der Bindungsreaktion werden SPM-Bilder als Funktion des Winkels θ mit Hilfe des CCD-Arrays 23 aufgenommen und mit der Bildanalyseeinrichtung 24 analysiert. Dadurch kann die Intensität des reflektierten Lichts in Abhängigkeit vom Reflexionswinkel räumlich differenziert für die Teilbereiche 38,39 beobachtet werden. Fig. 5 zeigt vier derartige Kurven, wobei die Intensität in willkürlichen (aber für alle Kurven gleichen) Einheiten gemittelt über Flächenelemente von jeweils 20µm x 20µm in den jeweiligen Teilbereichen gegen den Reflexionswinkel aufgetragen ist. Die einzelnen Kurven betreffen dabei folgende Fälle:
a) (offene Kreise): Matrixbereich in Streptavidin-freier Probenflüssigkeit,
b) (offene Dreiecke): Kristalline Bereiche in Streptavidin-freier Probenflüssigkeit,
c) (gefüllte Dreiecke): Kristalline Bereiche im Gleichgewicht mit Streptavidin-haltiger Probenflüssigkeit,
d) (gefüllte Kreise): Matrixbereich im Gleichgewicht mit Streptavidin-haltiger Probenflüssigkeit.

Die Kurven zeigen das für Beobachtungen der Oberflächenplasmonenresonanz typische Minimum beim Resonanzwinkel. Hier wird nahezu die vollständige eingestrahlte Energie zur Anregung von Plasmonen verbraucht. Das Anwachsen der Schichtdicke durch die Bindung des Streptavidins führt zu einer Verschiebung des Minimums. Dabei kann der Anteil der spezifischen Bindung durch Vergleich der Verschiebung in dem biotinreichen Teilbereich (Pfeil 45) mit der entsprechenden Verschiebung in dem biotinarmen Teilbereich (Pfeil 46) gegenüber dem Einfluß der unspezifischen Bindung differenziert werden.

Da die dargestellten Kurven den bekannten SPR-Abtastungen äquivalent sind und deswegen in üblicher Weise durch Fresnel-Rechnungen verglichen werden können, läßt sich die Dickenzunahme sogar quantitativ auswerten. Unter der Annahme, daß die beiden koexistierenden Phasen den gleichen Brechungsindex von n_{eff} = 1,5 haben, ergibt sich aus den Verschiebungen 45 und 46 für den stärker bindeaktiven fluid-amorphen Teilbereich 39 ein Dickenzuwachs von 3,4 nm, für den weniger bindeaktiven Teilbereich 38 ein Dickenzuwachs von 1,8 nm.

Bei der analytischen Untersuchung von Proben wird üblicherweise eine Kalibration durchgeführt, bei der eine physikalische Meßgröße des jeweiligen Verfahrens für eine oder mehrere Standardflüssigkeiten, in denen der Analyt in bekannter Konzentration enthalten ist, bestimmt wird. Bei der Erfindung kann als Meßgröße in diesem Sinne die Differenz der Verschiebungen des Minimums zwischen den Teilbereichen unterschiedlicher Bindeaktivität des ersten Bindungspartners verwendet werden. Es sind jedoch auch kompliziertere Algorithmen möglich, durch die die Meßsignale der verschiedenen Teilbereiche in Relation zueinander gesetzt und zu einer für die Kalibration geeigneten physikalischen Meßgröße verarbeitet werden.

Fig. 6 zeigt für die beiden Teilbereiche unterschiedlicher Bindeaktivität die zeitliche Veränderung des Resonanzwinkels, wobei wiederum die Kreise die Meßwerte des Bereiches mit hoher Biotin-Bindeaktivität und die Dreiecke die Meßwerte der Bereiche mit niedriger Biotin-Bindeaktivität bezeichnen. Dabei entspricht ein höherer Winkel einer höheren Schichtstärke.

Man erkennt, daß der amorphe Teilbereich mit hoher Bindeaktivität des fixierten Bindungspartners zunächst eine niedrigere Schichtstärke hat, diese aufgrund der spezifischen Bindung des Streptavidins dann aber schnell anwächst und nach etwa 0,5 Std. die Dicke der Teilbereiche mit niedriger Bindeaktivität des fixierten Bindungspartners übertrifft. Im weiteren Verlauf nähern sich die Kurven assymtotisch dem Gleichgewichtszustand. Dieses Verhalten läßt sich analytisch vorteilhaft nutzen. In der Anfangsphase ist die Kinetik der Bindung zwischen den beiden Teilbereichen besonders deutlich verschieden. Eine kinetische Messung der Dickenzunahme erlaubt deshalb eine besonders gute Differenzierung, d.h. man gewinnt ein störungsarmes Nutzsignal. Außerdem wird die Meßzeit erheblich reduziert, weil eine verhältnismäßig kurze kinetische Messung zu Beginn der spezifischen Bindungsreaktion ausreicht.

Damit ist gezeigt, daß sich die Bindeaktivitäten der Teilbereiche 38 und 39 sowohl hinsichtlich der maximalen Bindekapazität, d.h. der Menge des pro Flächeneinheit maximal gebundenen freien Bindungspartners Streptavidin, als auch hinsichtlich der Kinetik des Bindevorgangs unterscheiden. Auf welche Einflüsse diese Unterschiede im Beispielsfall zurückzuführen sind, ist nicht vollständig aufgeklärt. Es ist jedoch anzunehmen, daß die Konzentration des biotinylierten Lipids in den kristallinen Bereichen 38 geringer ist als in dem fluid-amorphen Teilbereich 39, und daß auch die sterische Zugänglichkeit in den kristallinen Teilbereichen durch die kristalline Pakkung erschwert ist.

Die Struktur des LB-Films, welcher bei dem anhand der Figuren 2 und 3 beschriebenen Labormuster verwendet wurde, ist unregelmäßig und verhältnismäßig kompliziert (vg1. Fig.4). Für die praktische Anwendung der Erfindung ist eine einfachere Struktur des Testbereichs bevorzugt. Beispielsweise ist in Fig. 7 ein Analyseelement 10' dargestellt, dessen Testbereich 13' eine Mehrzahl von streifenförmigen Teilbereichen aufweist, die mit 14' bzw. 15' bezeichnet sind. Die Teilbereiche 14a' bis 14c' bilden einen ersten Satz A, während die Teilbereiche 15a' bis 15c' einen zweiten Satz B von Teilbereichen bilden. Die Teilbereiche beider Sätze unterscheiden sich in der Bindeaktivität des trägerfixierten Bindungspartners.

Die in Fig. 7 dargestellte streifenförmige Struktur der Teilbereiche ist eine mögliche Ausführungsform einer eindimensional alternierenden Struktur. Durch eine solche Struktur wird im Vergleich zu einer zweidimensionalen Verteilung, wie sie in Fig. 4 dargestellt ist, die räumlich differenzierte Erfassung der Teilbereiche erleichtert. Im Falle der Auswertung mittels SPM kann beispielsweise statt des in Fig. 2 eingezeichneten CCD-Array eine lineare Anordnung lichtempfindlicher Elemente benutzt werden. Dabei wird statt der sphärischen Linse 22 zweckmäßigerweise eine Zylinderlinse zur Abbildung verwendet. Damit wird der Aufwand für.die Bildanalyse erheblich reduziert.

Auch zur Auswertung eindimensional alternierender Teilbereiche kann jedoch vorteilhaft eine zweidimensionale Anordnung lichtempfindlicher Elemente (also beispielsweise ein CCD-Array wie dargestellt, jedoch in Verbindung mit einer Zylinderlinse) verwendet werden. Dabei wird eine Zylinderlinse so angeordnet, daß die eindimensionale Struktur streifenförmig in der Bildebene abgebildet wird. Die zweite Dimension erlaubt dabei die Analyse der Winkelabhängigkeit der reflektierten Intensität ohne Bewegung des Primärstrahls.

Eine Anordnung zweier größerer Teilbereiche, wie sie in Fig. 1 dargestellt ist, läßt sich mit Hilfe der LB-Technik erzeugen, indem die Tragschicht 11 zweifach, von zwei gegenüberliegenden Kanten her, teilweise eingetaucht wird.

Allgemein kann der Testbereich mit den verschiedenen Teilbereichen jedoch auch in anderer Weise erzeugt werden, sofern nur sichergestellt ist, daß die Schichtstärke der den ersten Bindungspartner enthaltenden Schicht in den Teilbereichen so dünn ist, daß sich ihre Zunahme durch die spezifische Bindung mit dem freien Bindungspartner mit Hilfe des jeweiligen reflexionsoptischen Meßverfahrens mit ausreichender Genauigkeit auswerten läßt und solange die verschiedenen Teilbereiche sehr ähnliche Eigenschaften bezüglich unspezifischer Bindungen haben. Beispielsweise können Drucktechniken (insbesondere die Düsenstrahl-Drucktechnik (Ink-Jet)) oder Photomasken-Techniken verwendet werden.

Die Wahl des reflexionsoptischen Meßverfahrens hängt unter anderem von der Anordnung und Größe der Teilbereiche in dem Testbereich ab.

Im Falle der Oberflächenplasmonenresonanz genügt das übliche SPR-Verfahren, soweit die Teilbereiche so groß sind, daß sie getrennt mit einem Laserstrahl beleuchtet und die dabei gewonnenen Reflexionsintensitäten getrennt ausgewertet werden können. Besonders bevorzugt ist jedoch die SPM-Technik. Sie erlaubt eine sehr fein aufgelöste räumlich differenzierte Erfassung der Teilbereiche (Ortsauflösung auf 5 µm ist beim derzeitigen Stand der Technik möglich). Infolgedessen können die Teilbereiche sehr klein und verhältnismäßig kompliziert angeordnet sein, woraus sich Vorteile bezüglich der Handhabung und Herstellung des Analysesystems ergeben. Außerdem erfolgt die Auswertung der Meßsignale der Teilbereiche unter identischen Bedingungen, was zu einer Erhöhung der Meßgenauigkeit führt.

Sofern andere reflexionsoptische Meßverfahren eingesetzt werden, müssen sie eine ausreichende räumliche Differenzierung ermöglichen. Grundsätzlich sind die in dem einleitend zitierten Artikel von Sadowski erwähnten Techniken hierzu geeignet, wobei die dort erwähnte Brewsterwinkel-Reflektrometrie und Ellipsometrie bezüglich der Empfindlichkeit der Messung einer Dickenänderung mit der SPM vergleichbar sind, aber eine geringerere Ortsauflösung und die in dem Artikel erwähnten praktischen Nachteile haben. Die Messung der totalen inneren Reflexion an einem Lichtleiter, welche in der Arbeit von Sadowski ebenfalls näher erläutert wird, ist (ohne zusätzliche Modifikationen) nur für verhältnismäßig grobe Strukturen geeignet, weil die geringe Dämpfung des zugrundeliegenden physikalischen Mechanismus keine ausreichende laterale Auflösung ermöglicht. Durch Einführung künstlicher Dämpfungsmechanismen kann jedoch die laterale Auflösung so erhöht werden, daß auch ein solches Verfahren erfindungsgemäß eingesetzt werden kann.

## Patentansprüche

1. Analysesystem zur Bestimmung eines Analyten in einer fluiden Probe, insbesondere einer Körperflüssigkeit, mittels einer spezifischen Bindungsreaktion zwischen zwei bioaffinen Bindungspartner, von denen der eine trägerfixiert und der andere frei ist, umfassend
Analyseelemente (10) mit einem Testbereich (13), in dem der trägerfixierte Bindungspartner fixiert ist, wobei der Testbereich (13) räumlich getrennte Teilbereiche (14,15;38,39) mit unterschiedlicher Bindeaktivität des trägerfixierten Bindungspartners zu dem freien Bindungspartner aufweist,
und
ein Auswertegerät (1) mit einer reflexionsoptischen Meßeinrichtung (20) zur Erfassung einer aus der Bindung des freien Bindungspartners an den trägerfixierten Bindungspartner resultierenden Schichtdickenänderung und einer Auswerteeinheit (26) zur Ermittlung des Analysewertes aus der Schichtdickenänderung,
**dadurch gekennzeichnet,** daß
die reflexionsoptische Meßeinrichtung ein optisches Abbildungssystem mit einer Linse (22) aufweist, durch
das der Testbereich (13) in einer Bildebene abgebildet wird, wobei in der Bildebene lichtempfindliche Elemente zur räumlich differenzierten Erfassung der aus der Bindung des freien Bindungspartners an den trägerfixierten Bindungspartner resultierenden Schichtdickenänderung in den Teilbereichen angeordnet sind und
die Auswerteeinheit (26) eine Bildanalyseeinrichtung (24) mit einer Vergleichseinheit zum Vergleich der an den Teilbereichen gewonnenen Meßsignale aufweist.

2. Analysesystem nach Anspruch 1, **dadurch gekennzeichnet**, daß die Teilbereiche (14,15;38,39) unmittelbar aneinander angrenzen.

3. Analysesystem nach einem der vorhergehenden Ansprüche, **dadurch gekennzeichnet**, daß der Testbereich mindestens zwei Sätze von Teilbereichen (14a-14c,15a-15c) aufweist, welche jeweils einen oder mehrere Teilbereiche umfassen, wobei die Teilbereiche des gleichen Satzes die gleiche Bindeaktivität des trägerfixierten Bindungspartners aufweisen und die Teilbereiche verschiedener Sätze unterschiedliche Bindeaktivitäten des trägerfixierten Bindungspartners aufweisen und die Teilbereiche verschiedener Sätze in dem Testbereich alternierend angeordnet sind.

4. Analysesystem nach Anspruch 3, **dadurch gekennzeichnet**, daß die Teilbereiche (14a-14c,15a-15c) eindimensional alternierend angeordnet sind.

5. Analysesystem nach einem der vorhergehenden Ansprüche, **dadurch gekennzeichnet,** daß die Teilbereiche (38,39) mit der Langmuir-Blodgett-Technik erzeugt sind.

6. Analysesystem nach einem der vorhergehenden Ansprüche, **dadurch gekennzeichnet,** daß die Bindungspartner Streptavidin oder Avidin und Biotin sind.

7. Analysesystem nach einem der vorhergehenden Ansprüche, **dadurch gekennzeichnet,** daß es eine Plasmonenkoppleranordnung (30) einschließt und die reflexionsoptische Meßeinrichtung (20) zur Erfassung der Oberflächenplasmonenresonanz ausgebildet ist.

8. Analysesystem nach Anspruch 7, **dadurch gekennzeichnet,** daß die Meßeinrichtung (20) zur Oberflächenplasmonenmikroskopie ausgebildet ist.

9. Analysesystem nach Anspruch 1, **dadurch gekennzeichnet,** daß das Auswertegerät eine lineare Anordnung lichtempfindlicher Elemente aufweist.

10. Verfahren zur Bestimmung eines Analyten in einer fluiden Probe, insbesondere einer Körperflüssigkeit, mit Hilfe eines Analysesystems nach einem der Ansprüche 1 bis 9, **dadurch gekennzeichnet,** daß die Probe mit dem Testbereich derartig kontaktiert wird, daß sie mit den getrennten Teilbereichen in Kontakt steht, die an den Teilbereichen gewonnenen Meßsignale registriert und in Beziehung zueinander gesetzt werden und die Bindeaktivität des Analyten aus der Relation der Meßsignale der Teilbereiche ermittelt wird.

## Claims

1. Analytical system for determining an analyte in a fluid sample, in particular a body fluid, by means of a specific binding reaction between two binding partners having biological affinity to each other, one of which is carrier-fixed and the other free, comprising
analysis elements (10) with a test zone (13) in which the carrier-fixed binding partner is fixed, wherein the test zone (13) comprises spatially separated sub-zones (14, 15; 38, 39) with different binding activity of the carrier-fixed binding partner to the free binding partner,
and
a processing apparatus (1) with a reflection-optical measuring device (20) for recording a change in layer thickness resulting from the binding of the free binding partner to the carrier-fixed binding partner and with an evaluation unit (26) for determining the analysis value from the change in layer thickness,
**characterised in that**
the reflection-optical measuring device (20) comprises an optical imaging system with a lens (22) by which
the test zone (13) is imaged onto an image plane, where light-sensitive elements are located for the spatially differentiated recording of the thickness variation in the sub-zones resulting from the binding of the free binding partner to the carrier-fixed binding partner, and
the evaluation unit (26) comprises an image analyzing device (24) with a comparison unit for comparing the measurement signals obtained at the sub-zones.

2. Analytical system according to claim 1, **characterised in that** the sub-zones (14, 15; 38, 39) border one another directly.

3. Analytical system according to any one of the preceding claims, **characterised in that** the test zone comprises at least two sets of sub-zones (14a-14c, 15a-15c) each of which comprises one or more sub-zones, in which the sub-zones of the same set exhibit the same binding activity of the carrier-fixed binding partner and the sub-zones of different sets exhibit varying binding activities of the carrier-fixed binding partner and the sub-zones of different sets are arranged alternately in the test zone.

4. Analysis system according to claim 3, **characterised in that** the sub-zones (14a-14c, 15a-15c) are arranged alternately in one dimension.

5. Analysis system according to any one of the preceding claims, **characterised in that** the sub-zones (38, 39) are produced by the Langmuir-Blodgett technique.

6. Analysis system according to any one of the preceding claims, **characterised in that** the binding partners are streptavidin or avidin and biotin.

7. Analysis system according to any one of the preceding claims, **characterised in that** it includes a plasmon coupler arrangement (30) and the reflection-optical measuring device (20) is designed for recording the surface plasmon resonance.

8. Analysis system according to claim 7, **characterised in that** the measuring device (20) is designed for surface plasmon microscopy.

9. Analysis system according to claim 1, **characterised in that** the processing apparatus comprises a linear arrangement of light-sensitive elements.

10. Method for determining an analyte in a fluid sample, in particular a body fluid, by means of an analysis system according to any one of claims 1 - 9, **characterised in that** the sample is placed in contact with the test zone in such a way that it is in contact with the separated sub-zones, the measurement signals obtained at the sub-zones are recorded and related to one another, and the binding activity of the analyte is determined from the relationship between the measurement signals of the sub-zones.

## Revendications

1. Système d'analyse pour la détermination d'un analyte dans un échantillon fluide, en particulier un liquide corporel, au moyen d'une réaction de liaison spécifique entre deux partenaires de liaison par affinité biologique, dont l'un est fixé à un porteur et l'autre est libre, comprenant des éléments analytiques (10) avec un espace de test (13),
dans lequel est fixé le partenaire de liaison fixé au porteur, dans lequel l'espace de test (13) est constitué de sections séparées dans spatialement (14,15;38,39) avec une activité de liaison différente du partenaire de liaison fixé au porteur envers le partenaire de liaison libre,
et
un appareil d'évaluation (1) avec un dispositif de mesure à réflexion optique (20) pour l'acquisition d'une modification des épaisseurs de couche résultant d'une liaison entre le partenaire de liaison libre et le partenaire de liaison fixé au porteur et avec une unité d'évaluation (26) pour la détermination du résultat d'analyse de la modification des épaisseurs de couche, caractérisé en ce que,
le dispositif de mesure à réflexion optique est constitué d'un système de projection optique avec une lentille (22), au moyen duquel l'image de l'espace de test (13) est projetée sur un plan focal, dans lequel des éléments photosensibles sont disposés dans le plan focal pour la différenciation spatiale de la modification des épaisseurs de couche dans les sections résultant de la liaison entre le partenaire de liaison libre et le partenaire de liaison fixé au porteur et l'unité d'évaluation (26) présente un dispositif d'analyse d'image (24) avec une unité de comparaison pour la comparaison des signaux de mesure extraits des sections.

2. Système d'analyse selon la revendication 1, caractérisé en ce que les sections (14,15;38,39) sont directement adjacentes les unes aux autres.

3. Système d'analyse selon l'une quelconque des revendications précédentes, caractérisé en ce que l'espace de test comprend au moins deux jeux de sections(14a à 14c, 15a à 15c), lesquels comprennent chacun une ou plusieurs sections, dans lequel les sections du même jeu présentent la même activité de liaison du partenaire de liaison fixé au porteur et les sections de différents jeux présentent des activités de liaison différentes du partenaire de liaison fixé au porteur et les sections de différents jeux sont disposées alternativement dans l'espace de test.

4. Système d'analyse selon la revendication 3, caractérisé en ce que les espaces de test (14a à 14c,15a à 15c) sont disposés alternativement dans une dimension.

5. Système d'analyse selon l'une quelconque des revendications précédentes, caractérisé en ce que les espaces de test (38,39) sont créés à l'aide de la technique de Langmuir-Blodgett.

6. Système d'analyse selon l'une quelconque des revendications précédentes, caractérisé en ce que les partenaires de liaison sont de la streptavidine ou de l'avidine et de la biotine.

7. Système d'analyse selon l'une quelconque des revendications précédentes, caractérisé en ce qu'il renferme une disposition de coupleur (30) de plasmons et que le dispositif de mesure par réflexion optique (20) est prévu pour la détection à résonance de plasmons superficiels.

8. Système d'analyse selon la revendication 7, caractérisé en ce que le dispositif de mesure (20) est prévu pour la microscopie de plasmons superficiels.

9. Système d'analyse selon la revendication 1, caractérisé en ce que l'appareil d'évaluation est constitué d'une configuration linéaire d'éléments photosensibles.

10. Procédé pour la détermination d'un analyte dans un échantillon fluide, particulièrement un liquide corporel, à l'aide d'un système d'analyse selon l'une quelconque des revendications 1 à 9, caractérisé en ce que l'on met en contact l'échantillon avec l'espace de test de telle façon qu'il reste en contact avec les sections séparées, que l'on enregistre les signaux de mesure extraits et qu'on les met en correspondance l'un avec l'autre et que l'on détermine l'activité de liaison de l'analyte à partir de la relation des signaux de mesure provenant des sections.
